# EUROPÄISCHE PATENTSCHRIFT

(11) **EP 3 469 057 B1**
(45) Veröffentlichungstag und Bekanntmachung des Hinweises auf die Patenterteilung: **05.08.2020**
(21) Anmeldenummer: 17729440.2
(22) Anmeldetag: 07.06.2017
(51) Int. Cl.: C11D 3/40, C11D 3/50, C11D 17/00, E03D 9/03

(54) **VORRICHTUNG MIT REINIGUNGSMITTEL FÜR DEN TOILETTENBEREICH**
DEVICE HAVING A CLEANING AGENT FOR THE TOILET AREA
DISPOSITIF POURVU D'UN AGENT DE NETTOYAGE POUR LE DOMAINE DES TOILETTES

(30) Priorität: 08.06.2016 DE 102016110584
(43) Veröffentlichungstag der Anmeldung: 17.04.2019
(73) Patentinhaber: Buck-Chemie GmbH, 71083 Herrenberg (DE)
(72) Erfinder: LEIPOLD, Dr. Joachim, 72760 Reutlingen (DE); FRITZ, Matthias, 72810 Gomaringen (DE)
(74) Vertreter: Mammel und Maser
(86) Internationale Anmeldenummer: PCT/EP2017/063763
(87) Internationale Veröffentlichungsnummer: WO 2017/211857

(56) Entgegenhaltungen:
- EP-A1- 0 999 263
- EP-A1- 1 418 225
- EP-A2- 2 806 074

## Beschreibung

Die vorliegende Erfindung betrifft eine Vorrichtung mit Reinigungsmittel zur Befestigung am Rand einer Toilette, wobei das Reinigungsmittel stückförmig ist und eine Reinigungsmittelformkörperphase und eine Gelphase aufweist, sowie ein Herstellungsverfahren.

Ein Großteil der bekannten stückförmigen Mittel sind so genannte "Rimblocks", die in einem Behältnis, insbesondere einem Körbchen oder käfigartigem Behälter, am Rand ("Rim") der Toilette befestigt werden. Das in dem Behältnis befindliche Mittel wird bei jedem Spülvorgang von dem Spülwasser überströmt. Hierdurch wird bei jeder Spülung ein geringer Anteil des Reinigungsmittels unter Freisetzung von Tensiden, Duftstoffen etc. aufgelöst, wodurch dann die gewünschte Reinigung des Toilettenbeckens und Toilettensumpfs und die gewünschte Beduftung erzielt werden.

Die bekannten Rimblocks verbrauchen sich ausschließlich durch den Spülstrom des Wassers, und die Duftstofffreigabe ist von der Spülung der Toilette abhängig. Die Raumbeduftung wird bei den Rimblocks im Wesentlichen dadurch erzielt, dass die Duftstoffe durch das Spülwasser herausgespült werden, in den Toilettensumpf gelangen und ein Teil der Duftstoffe in dem Toilettensumpf verdampft.

Aus der DE 197 10 635 A1 sind stückförmige Toilettenreinigungsmittel bekannt, die Gelbildner, Lösemittel und Duftstoffe umfassen und die bei nur geringer Duftstoffdosierung eine ausreichende und permanente Raumbeduftung auch dann ermöglichen, wenn keine Toilettenspülung erfolgt.

Aus der EP 0 999 263 A1 sind stückförmige Reinigungsmittel für den Toilettenbereich, in welchen verschiedene unterschiedliche Farbzentren verteilt sind, bekannt.

Die EP 1 418 225 A1 lehrt stückförmige Reinigungsmittel mit einer Gelphase, die auch angefärbt sein kann.

Aus der EP 2 806 074 A1 sind Haltevorrichtungen mit Sichtfenster für Spültoiletten bekannt.

Ein Toilettenreinigungsmittel, das sowohl eine Reinigungsmittelformkörperphase als auch eine wasserlösliche Gelphase mit einem Duftstoff umfasst, ist aus der EP 2 374 483 B1 bekannt. Mit diesem Mittel wird sowohl die gewünschte Reinigungswirkung erzielt, als auch bei geringer Duftstoffdosierung eine ausreichende und permanente Raumbeduftung selbst dann ermöglicht, wenn keine Toilettenspülung erfolgt.

Zur Herstellung solcher Toilettenreinigungsmittel wird ein Reinigungsmittelformkörper mit Öffnungen oder Vertiefungen hergestellt, in die die flüssige heiße Gelphase eingefüllt wird. Bei den Aussparungen kann es sich beispielsweise um Rillen in der Oberfläche des Reinigungsmittelformkörpers handeln. Auch kann der Reinigungsmittelformkörper in Form eines hohlen Rohres extrudiert werden, wobei dann in den Innenraum die flüssige Gelphase eingefüllt wird. Nach dem Erkalten wird der Strang dann in einzelne Reinigungsmittelscheiben, die außen eine Reinigungsmittelformkörperphase und innen eine Gelphase aufweisen, geschnitten.

Die EP 1 553 162 A1 lehrt ein Reinigungsmittel mit einer Gelphase auf der Basis von vorzugsweise wasserunlöslichen Polyamidharzen und einer Reinigungsmittelformkörperphase.

Toilettenreinigungsmittel mit Gel- und Reinigungsmittelformkörperphase weisen häufig eine weiße oder farbige Reinigungsmittelformkörperphase und eine andersfarbige Gelphase auf.

Die Ansprüche der Verbraucher an die optische Wirkung von Reinigungs- und Beduftungsmitteln für den Sanitärbereich steigen stetig. Die Mittel sollen nicht nur reinigen und den Raum beduften, sondern gleichzeitig in einem modernen Design optisch ansprechend gestaltet sein.

Um den Toilettenreinigungsmitteln ein ansprechendes Aussehen zu geben, werden diese Mittel auch mit einer eingefärbten Gelphase angeboten. Auch dreifarbige Reinigungsmittel mit zwei unterschiedlich farbigen Gelphasen in rot und blau und einer Reinigungsmittelformkörperphase in weiß sind beispielsweis aus dem EU-Geschmacksmuster 000748975-0009 bekannt.

Neben diesen Toilettenreinigungsmitteln mit verschiedenen Farben, die in entsprechenden Körbchen angeboten werden, sind in den letzten Jahren auch WC-Körbchen bekannt geworden, die mehrere Reinigungsmittel in verschiedenen Farben enthalten, die nicht nur einem optisch ansprechenden Äußeren dienen, sondern auch dem Verbraucher unterschiedliche Inhaltsstoffe und Funktionen visualisieren.

Die einzelnen Reinigungsmittel können beispielsweise scheiben-, kugel- oder würfelförmig sein und werden häufig in zwei wechselnden Farben, beispielsweise in Form von einzelnen pinken und blauen Kugeln (in der Farbabfolge pink-blau-pink-blau) gemäß dem EU-Geschmacksmuster 001973579-0003 oder in Form von verschiedenfarbigen Scheiben angeboten.

Meist werden solche verschiedenfarbigen einzelnen Reinigungsmittelkörper, die in den Körbchen bevorratet werden, aus pulverförmigen Ausgangsstoffen extrudiert, was jedoch aufgrund der offenporigen und rauen Oberfläche zu hohen Streukoeffizienten der Reinigungsmittelformkörperformulierung und damit zu blasseren Farben führt.

Solche WC-Körbchen mit den verschiedenfarbigen einzelnen Mitteln sehen zwar optisch ansprechend aus, allerdings ist die Herstellung der einzelnen verschiedenfarbigen Reinigungsmitteln und die Befüllung der Körbchen mit den einzelnen verschiedenfarbigen Reinigungsmitteln aufwendig.

Die Aufgabe der vorliegenden Erfindung besteht darin, ein WC-Körbchen mit einem Reinigungsmittel anzugeben, das den Wünschen des Verbrauchers an ein optisch ansprechendes, besonderes Design gerecht wird, einfach herstellbar ist und aufgrund von Verschiedenfarbigkeit zur Visualisierung unterschiedlicher Funktionen geeignet ist.

Diese Aufgabe wird erfindungsgemäß dadurch gelöst, dass das Behältnis, das das stückförmige Reinigungsmittel umfasst, wenigstens zwei in Richtung des Inneren der Toilettenschüssel weisende, voneinander beabstandete Sichtöffnungen aufweist und das Reinigungsmittel eine Reinigungsmittelformköperphase und eine Gelphase aufweist, wobei sich die Farbe der Gelphase von der einen Seite des Reinigungsmittels zu der anderen Seite des Reinigungsmittels im Wesentlichen kontinuierlich ändert und die Gelphase des Reinigungsmittels durch die Sichtöffnungen des Behältnisses sichtbar ist, so dass sich der Farbeindruck der Gelphase hinter der ersten Sichtöffnung von dem Farbeindruck der Gelphase hinter der zweiten Sichtöffnung unterscheidet.

Erfindungsgemäß kann somit mit einem einzigen Reinigungsmittel in einem Behältnis, das insbesondere ein WC-Körbchen ist, dadurch, dass das Reinigungsmittel seine Farbe in der Gelphase von der einen Seite zu der anderen Seite des Mittels im Wesentlichen kontinuierlich ändert und sich das Mittel in dem Behältnis wenigstens über den gesamten Bereich der Öffnungen der beiden voneinander beabstandeten Sichtöffnungen erstreckt, in einer jeden Sichtöffnung ein anderer Farbeindruck erzeugt und damit auch unterschiedliche Funktionen des Mittels visualisiert werden.

Dadurch, dass die Erzeugung eines unterschiedlichen Farbeindrucks mit nur einem Mittel ermöglicht wird, kann die Herstellung und Lagerung von unterschiedlich farbigen Mitteln und deren Sortieren und Einfüllen in Einzelbereiche des Behältnisses entfallen.

Erfindungsgemäß können somit mit nur einem erfindungsgemäßen Mittel und einem erfindungsgemäßen Körbchen verschiedene Farbeindrücke erzeugt, ein ansprechendes, farbintensives "frisches" Design bereitgestellt und eine Visualisierung verschiedener Funktionen erreicht werden.

Ein weiterer Vorteil der Erfindung ist, dass das dortige Mittel nicht nur einfach herstellbar ist, sondern kostengünstig auch brillante Farben und Farbwechsel ermöglicht.

Dadurch, dass erfindungsgemäß die Gelphase eingefärbt ist und nicht die Reinigungsmittelformkörperphase, die im Allgemeinen weiß ist, ist die farbige Phase transparent und wenig streuend. Da eine transparente Gelphase vorliegt und keine streuende Phase, ist der Farbeindruck im Vergleich zu den eingefärbten Reinigungsmittelformkörpern brillanter und kräftiger. Im Rahmen der vorliegenden Erfindung ist eine Gelphase transparent, wenn man durch eine Gelplatte mit einer Schichtdicke von 1 cm noch eine Schrift mit einer Höhe von 7 Pixeln, die mit einem HP Laserjet 2100 erstellt wurde, lesen kann.

Zudem lässt sich ein harmonischer brillanter Farbverlauf in der Gelphase von einer Farbe zu einer anderen Farbe auf einfache Weise herstellen, derart, dass die Reinigungsmittelformkörperphase im Wesentlichen U- oder wannenförmig ausgebildet ist und einen Aufnahmeraum für die Gelphase aufweist, wobei nahe dem ersten Schenkel der U-förmigen Reinigungsmittelformkörperphase flüssige Gelphase einer ersten Farbe und nahe dem zweiten Schenkel der U-förmigen Reinigungsmittelformkörperphase flüssige Gelphase einer zweiten Farbe eingefüllt wird. Während des Befüllens vermischen sich die beiden verschiedenfarbigen Gelphasen teilweise, so dass letztendlich ein kontinuierlicher Farbverlauf von der Innenseite des ersten Schenkels der U-förmigen Reinigungsmittelformkörperphase mit einer ersten Farbe zu der Innenseite des zweiten Schenkels der U-förmigen Reinigungsmittelformkörperphase mit einer zweiten Farbe erhalten wird.

Dabei sollte die Viskosität der beiden verschiedenfarbigen Gelphasen beim Einfüllen einerseits so gering sein, dass die beiden Gelphasen teilweise ineinanderfließen und sich teilweise vermischen, den gesamten Aufnahmeraum ausfüllen und beim Aushärten eine homogene glatte Oberfläche bilden und andererseits so hoch, dass keine vollständige Durchmischung der beiden Farben über die gesamte Breite der Gelphase erfolgt.

Vorzugsweise ist das Reinigungsmittel im Wesentlichen quaderförmig, wobei die Reinigungsmittelformkörperphase einen U-förmigen Aufnahmeraum für die Gelphase aufweist und die Reinigungsmittelformkörperphase drei Seiten des Quaders bildet und die dem Boden gegenüberliegende Sichtseite des Reinigungsmittels im Wesentlichen durch die Gelphase gebildet wird, wobei diese im Bereich zwischen den beiden Schenkeln der Gelphase den Farbverlauf aufweist und die Sichtseite seitlich durch die Enden der beiden Schenkel der Reinigungsmittelformkörperphase begrenzt ist.

Dadurch, dass sich unterhalb der transparenten Gelphase noch die nicht transparente, streuende Reinigungsmittelformkörperphase befindet, die im Allgemeinen weiß ist und das Licht, das die Gelschicht durchquert hat, wieder reflektiert wird, durchquert das Licht die doppelte Schichtdicke der Gelphase, d.h. der Farbeindruck - und damit auch der Eindruck der Farbänderung - ist durch diesen Effekt gegenüber einem einfach transparenten System verstärkt. Insofern ist es möglich, auch bei dünnen Gelschichten von 1 bis 10 mm, vorzugsweise 2 bis 6 mm, und einem Farbwechsel über die Breite der Gelphase, z.B. von gelb nach rot, auch bei geringer Schichtdicke der Gelphase, einen deutlich unterschiedlichen Farbeindruck des Mittels hinter der einen und der anderen Öffnung zu erzielen.

Da sich die Farbe von der einen Seite der Gelphase zu der anderen Seite der Gelphase im Wesentlichen kontinuierlich ändert, ändert sich auch bei wenigstens einer Wellenlänge im sichtbaren Spektralbereich über die Breite der Gelphase die Extinktion.

Unter _{"}im Wesentlichen kontinuierliche Änderung der Farbe bzw. der Extinktion" ist eine Farbänderung zu verstehen, die sich über die Breite der Gelphase ergibt, wenn wenigstens zwei verschiedenfarbige Gelphasen an unterschiedlichen Orten in Bezug auf die Breite der Gelphase in den Aufnahmeraum der Reinigungsmittelformkörperphase eingefüllt werden. Dieses Merkmal ist auch dann erfüllt, wenn sich im Bereich des einen und/oder auch des anderen Schenkels die Farbe zunächst nicht oder wenig ändert und sich der Bereich des kontinuierlichen Farbumschlags von der einen Farbe in die andere Farbe eher auf den Mittenbereich der Gelphase beschränkt. Dieses Merkmal ist auch dann erfüllt, wenn nur eine Gelphase farbig und die andere transparent ist.

Unter "Breite" der Gelphase wird dabei der Abstand zwischen den beiden Schenkeln der U-förmigen Reinigungsmittelformkörperphase verstanden.

Selbstverständlich können in den Aufnahmeraum der Reinigungsmittelformkörperphase auch drei, vier oder mehr verschiedenfarbige Gelphasen eingefüllt werden, sofern der Aufnahmeraum genügend breit ist. In einem solchen Fall kann beispielsweise ein Reinigungsmittel mit einem brillanten, optisch ansprechenden Farbverlauf von rot über gelb über grün nach blau nebst den sich jeweils zwischen zwei Farben ergebenden Zwischenfarben erzielt werden. Die weiteren Düsen werden in diesem Fall zwischen den beiden nahe der Schenkel der Reinigungsmittelformkörperphase befindlichen Düsen platziert.

Wird ein solches vier Grundfarben und eine ungeheure Vielzahl von Zwischenfarben aufweisendes Mittel mit hinreichender Breite in ein WC-Körbchen mit vier Sichtöffnungen eingefüllt, so kann ein Körbchen mit vier unterschiedlich aussehenden Reinigungsmittel(bereichen) mit brillanten Farben bereitgestellt werden.

Auf der Grundlage der wenigstens zwei Gelphasen mit unterschiedlichen Farben, die nach der Extrusion der Reinigungsmittelformkörperphase gleich in den Aufnahmeraum eingefüllt werden, lassen sich auch weitere interessante Farbverläufe und Effekte erzielen, beispielsweise dadurch, dass die Gelphase unterschiedliche Temperaturen aufweist oder die Viskositäten der wenigstens zwei Gelphasen unterschiedlich sind, so dass die eine Gelphase mehr in die andere "hineinverläuft" als umgekehrt. Durch unterschiedliche Dichten der beiden Gelphasen "schwimmt" die leichtere Gelphase in einem gewissen Maße eher auf der Oberfläche auf, was der Gelphase optisch zusätzlich eine "Tiefe" verleihen kann. Weitere optische Effekte sind durch unterschiedliche Volumenströme, Veränderungen der Stranggeschwindigkeit, Bewegungen der Düsen während des Spritzens der Gelphasen in den Aufnahmeraum oder die Geometrie der Reinigungsmittelformkörperphase erzielbar.

Weiterhin können die Gelphase(n) auch mit farbigen Pigmenten oder anderen dekorativen Zusätzen ausgerüstet werden. Eine Vielzahl von Gestaltungsmöglichkeiten bietet beispielsweise das Programm der Firma BASF Chione® oder der Firma Deko Stones an.

### I. Die Gelphase

Die Gesamtgelphasen enthalten Gelbildner, Duftstoffe und Lösungsmittel.

Als Gelbildner können beispielsweise Metallseifen der höheren Fettsäuren, insbesondere die entsprechenden Alkalisalze wie Natriumstearat oder Natriumoleat, eingesetzt werden. Zudem sind auch andere anionische Tenside einsetzbar wie beispielsweise Alkylsulfate, Alkylethersulfate, Alkylethersulfonate, ethoxylierte Arylalkylsulfate, α-Olefinsulfonate, β-Alkoxyalkansulfonate, Alkylarylsulfonate, Alkylmonoglyceridsulfate, Alkylmonoglyceridsulfonate, Alkylcarbonate, Alkylethercarboxylate, Alkylphosphate, Alkyletherphosphate, Sulfosuccinate, Sarcosinate, Octoxynol- oder Nonoxynolphosphate, Taurinate, Polyoxyethylen-Fettsäureamidsulfate, Isethionate, anionische Derivate der Polyglycolethoxylate und/oder Kombination daraus, sofern diese Gele bilden. Ebenfalls ist möglich, als Gelbildner nichtionische Tenside, wie beispielsweise Ethylenoxid-Propylenoxid-(EO/PO)-Blockpolymere, Glycerinethoxylate, partielle Glycerinethoxylate, Polyglycolethoxylate, Polyalkoxyalkane, zum Beispiel ein Gemisch aus Alkyl-(C20,C22)-ethoxylat mit 35 EO, Alkyl-(C22)-ethoxylat mit 35 EO oder Alkyl-(C16,C18)-ethoxylat mit 30 EO, natürliche Polymere ("gums") wie Gummi Arabicum, Guar-Gum, Agar Agar, Pektine, Gelatine, Stärke, Sorbitol, chemisch modifizierte oder derivatisierte natürliche _{"}gums" wie Carboxymethylcellulose, Methylcellulose, Hydroxypropylmethylcellulose, Xanthan, Sorbitolderivate wie Dibenzylidensorbitol und deren Derivate, Stärkederivate wie Carboxymethylstärke, Hydroxyethylstärke, mikrobiell fermentierte _{"}gums" wie Dextran, Polysaccharid dB-1459 und Gelbildner wie Methoxypectin, Propylenglycolalginat, Triethanolaminalginat, Carboxymethyl-Guar-Gum etc., und/oder Gemische daraus einzusetzen, sofern diese Gele bilden.

Des Weiteren können auch Polymere als Gelbildner eingesetzt werden wie beispielsweise Polyalkoxylate (Polyacrylsäure, Polyacrylsäurederivate, Polymalinate, etc.), Polystyrol-Ethylen-Butylen-Styrol-Copolymere (SEBS), Styrol-Ethylen-Ethylen-Propylen-Styrol-Copolymere (SEEPS), Styrol-Ethylen-Propylen-Styrol-Copolymere (SEPS), Styrol-Ethylen-Propylen-Copolymere (SEP) und/oder Kombinationen daraus, sofern diese Gele bilden.

Ebenso können kationische Tenside als Gelbildner eingesetzt werden, wie beispielsweise Salze von aminierten Fettsäuren, Alkylpyridiniumsalze, quartäre Ammoniumsalze, quartäre ethoxylierte Amine, alkylierte Ammoniumsalze, polymere Ammoniumsalze, arylierte Ammoniumsalze, alkylierte und arylierte Ammoniumsalze, quartäre Polydimethylsiloxane, quartäre Silane und/oder Kombination daraus, sofern diese Gele bilden.

Ebenfalls ist der Einsatz von hydrophoben Gelbildnern wie beispielsweise Polyamidharzen, insbesondere Ester-terminerten Polyamid-Harzen, die beispielsweise in der US 6 268 466 beschrieben sind, möglich. Diese Harze können mit hydrophoben Lösemitteln Gele bilden, so dass die Gelbildung mit den Parfümölen ohne Zusatz von Lösemittel erfolgen kann, wie in der EP 1 553 162 beschrieben ist. Bei diesen Gelbildnern kann die Duftstoffkonzentration bis zu 60 Gew.% der Gelphase betragen.

Der Anteil des Gelbildners in der Gelphase hängt von dem jeweils verwendeten Gelbildner ab. Werden Metallseifen als Gelbildner eingesetzt, so beträgt deren gewichtsprozentualer Anteil zwischen 0,5 Gew.% und 25 Gew.% und vorzugsweise zwischen 1,0 Gew.% und 20 Gew.% und besonders bevorzugt zwischen 1,5 Gew.% und 15 Gew.%.

Bei Einsatz von Polyalkoxyalkanen als Gelbildner beträgt deren Anteil zwischen 10 Gew.% und 40 Gew.%, vorzugsweise zwischen 15 Gew.% und 35 Gew.%, beim Einsatz von natürlichen oder synthetischen _{"}gums" werden vorzugsweise zwischen 0,1 Gew.% und 10 Gew.% eingesetzt.

Bei den in der Gelphase enthaltenen Duftstoffen kann es sich um Reinstoffe oder um Duftstoffgemische, vorzugsweise ein Parfümöl oder Parfümölgemisch, handeln. Unter einem Duftstoff wird im Rahmen der vorliegenden Erfindung ein dem Geruchssinn angenehmer, chemischer Stoff oder ein entsprechendes Stoffgemisch von Riechstoffen verstanden. Kein Duftstoff im Sinne der vorliegenden Erfindung ist ein flüchtiger, aber unangenehm riechender Stoff. Der Anteil von Duftstoffen in der Gelphase beträgt vorzugsweise wenigstens 2 Gew.%, vorzugsweise zwischen 6 Gew.% und 50 Gew.% und besonders bevorzugt zwischen 15 Gew.% und 35 Gew.%.

Ein weiterer wesentlicher Bestandteil der Gelphase ist das Lösemittel. Prinzipiell kann ein organisches Lösemittel, Wasser oder deren Gemische als Lösemittel eingesetzt werden. Neben Wasser sind insbesondere Alkylenglycolalkylether wie beispielsweise Propylenglycol-n-Buthylether, Dipropylenglycol-Methylether oder deren Gemisch als Lösemittel geeignet. Auch können als Lösemittel C₃ - C₅ Glykole, Propylencarbonate, C₂ - C₄ Alkohole oder Polyalkylenglycole, vorzugsweise mit 200 - 600 Alkylenglycoleinheiten, Polyalkohole, (2)-Methyl-l,3-Propandiol, auch in Mischungen anderer Lösemittel, verwendet werden. Das beziehungsweise die Lösemittel sind im Allgemeinen polare Lösemittel, die die Gelbildner lösen. Ihr prozentualer Anteil beträgt zwischen 5 Gew.% und 70 Gew.%.

Bei dem unpolaren Gelbildner aus der Klasse der Polyamidharze und andere eher unpolare Gelbildner werden vorzugsweise unpolare Lösemittel bzw. auch Parfümöle als Lösemittel verwendet.

Der Gesamtgehalt an Lösemitteln in der Gelphase wird durch die für die Gelbildung mit dem jeweiligen Gelbildner erforderliche Lösemittelmenge bestimmt. Der Gesamtlösemittelgehalt der Gelphase schließt das in anderen Bestandteilen enthaltene Wasser, beispielsweise den Wassergehalt des zugesetzten Schäumers, mit ein. Wird als Gelbildner eine Metallseife eingesetzt, so beträgt der Gesamtlösemittelanteil wenigstens 10 Gew.%, vorzugsweise mehr als 20 Gew.%. Als Lösemittel kann beispielsweise 0 Gew.% bis 40 Gew.% Wasser zusammen mit 0 Gew.% bis 50 Gew.% organischem Lösungsmittel, beispielsweise 20 Gew.% bis 25 Gew.% Wasser und 30 Gew.% bis 40 Gew.% organisches Lösemittel, vorzugsweise aus der Gruppe der Alkylenglycolalkylether, Polyalkohole oder Parfümöle oder deren Mischung, eingesetzt werden.

Sofern die Duftstoffe flüssig sind, sind die Dufstoffanteile auch in den Lösemittelanteilen enthalten.

Die Farbstoffe in den Gelphasen können aus der Gruppe der Säure-, Beizen-, Dispersions-, Natur-, Lebensmittel-, Leder-, Direkt-, Schwefel-, Küpen-, Entwicklungs-, Reaktiv-, Lösemittelfarbstoffe, der basischen oder organischen Farbstoffe, der Pigmente, optischen Aufheller, der Zwischenprodukte zur Farbenentwicklung und der Pigmente ausgewählt werden.

Dabei sollte der Anteil des Farbstoffs in den Gelphasen wenigstens 0 Gew.% bis 10 Gew.%, vorzugsweise 0,001 Gew.% bis 5 Gew.% und besonders bevorzugt zwischen 0,01 Gew.% und 0,1 Gew.% betragen.

Die Gelphase kann zusätzlich einen oder mehrere Schäumer wie beispielsweise Betaine, alkoxylierte Alkylethersulfate oder Lactobionsäurederivate umfassen, wie beispielsweise Fettsäureamidopropyl-Betain mit einem C5 - C21-Fettsäureanteil wie beispielsweise Kokosamidopropylbetain, Alkali- oder Ammoniumsalze der Laurylethersulfate mit 1 bis 5 EO, Lactobionococylamid, Lactobionooleyl-amid, Lactobionotalgamid etc. oder deren Mischungen. Diese Schäumer lassen sich außerordentlich gut in die Gelphase einbringen. Besonders bevorzugt ist der Einsatz der flüssigen Superschäumer, nämlich der Betaine und der Laurylethersulfate. Sofern zusätzlich in der Gelphase ein Schäumer eingesetzt wird, beträgt dessen Anteil vorzugsweise bis zu 10 Gew.%, besonders bevorzugt 3 Gew.% bis 7 Gew.%.

Falls gewünscht, kann die Gelphase weiterhin Di-, Oligo- oder Polyhydroxyverbindungen oder deren Ether wie Glykol, 1,2- oder 1,3-Dihydroxypropan, 1,2-, 1,3-, 2,3- oder 1,4-Dihydroxybutan, die Isomere des Dihydroxyisobutans, Dihydroxypentans etc., Glycerin, Pentaerythrit, Penta- oder Hexahydroxyverbindungen oder Polyhydroxyether wie Polymethylen- oder Polypropylenglykol in einem Anteil von bis zu 20 Gew.% umfassen, um die Bildung schwerlöslicher Rückstände durch Austrocknung des Gels zu verhindern, den Auflösevorgang zu beschleunigen und eine ansehnlich glatte Oberfläche der Gelphase zu erreichen. Ebenfalls können der Gelphase zusätzlich Konservierungsmittel, z.B. Hydroxybenzoesäurealkylester wie Hydroxybenzoesäuremethyl- oder -propylester, zugesetzt werden, im Allgemeinen zwischen 0 Gew.% und 1 Gew.%.

Zur Steuerung der Auflösegeschwindigkeit können der Gelphase zusätzlich Hydrophobiermittel wie beispielsweise Kokosfettsäuremonoethanolamid oder andere bekannte Hydrophobiermittel zugegeben werden, bevorzugt sind 0 Gew.% bis 10 Gew.% Hydrophobiermittel einzusetzen. Prinzipiell tragen auch die in der Gelphase enthaltenen hydrophoben Parfümöle zu einer Verminderung der Auflösegeschwindigkeit bei.

Weiterhin kann die Gelphase weitere übliche Bestandteile wie Tenside, insbesondere schäumende Tenside, die kein allzu hohes Netzvermögen aufweisen, so dass sie sich nicht an die freizusetzenden Duftstoffe binden, Desinfektionsmittel oder auch Salze zur Steuerung der Auflösegeschwindigkeit umfassen.

Vorzugsweise sollte der Schmelzpunkt der Gelphase - um eine formstabile Lagerung und den Transport des Mittels zu gewährleisten - wenigstens 40 °C, bevorzugt wenigstens 50 °C betragen.

Damit eine Gelphase einer bestimmten Farbe eine bestimmte Funktion visualisiert, kann beispielsweise die rote Gelphase zusätzlich Schäumer und die gelbe Gelphase zusätzlich Desinfektionsmittel wie quartäre Ammoniumverbindungen umfassen.

Die Viskosität der Gelphase, gemessen mit einem Haake VT 500, einem Messbecher MV, einem Drehkörper MV II, einem Thermostat Haake DC 50, in einem Wasserbad Haake V15 mit einer Temperatur von 85 °C, sollte während des Einfüllvorgangs in den Aufnahmeraum der Reinigungsmittelformkörperphase im Allgemeinen zwischen 2 mPa·s und 2.000 mPa·s, vorzugsweise zwischen 3 mPa·s und 500 mPa·s und besonders bevorzugt zwischen 4 mPa·s und 20 mPa·s betragen.

### II. Die Reinigungsmittelformkörperphase

Unter einem Reinigungsmittelformkörper wird ein fester Körper verstanden, der vorzugsweise durch Extrusion, Verpressen oder Erstarren aus einer Schmelze entsprechend den üblichen Herstellungsverfahren von Rimblocks hergestellt wird.

Der Reinigungsmittelformkörper, der wenigstens 10 % Tenside enthält, dient der Reinigung und kann einen Duftstoff zur Beduftung der Toilette enthalten.

Der Reinigungsmittelformkörper umfasst Tenside, um die gewünschte Reinigungswirkung zu erzielen. Diese Tenside sollten zur Erzielung der gewünschten Reinigung gut netzende Tenside, somit vorzugsweise anionische oder nichtionische Tenside, sein. Prinzipiell sind alle bekannten anionischen Tenside geeignet. Vorzugsweise werden als anionische Tenside Alkylbenzolsulfonate, Alkylsulfate, Fettalkoholsulfate und Fettalkoholethersulfate eingesetzt. Vorzugsweise umfasst die Alkylgruppe oder der Fettsäurebestandteil zwischen 8 und 18 Kohlenstoffatome. Als Alkylbenzolsulfonat kann beispielsweise Natrium-alkyl-(C10-C13)-benzolsulfonat eingesetzt werden.

Der Einsatz von amphoteren Tensiden ist ebenfalls möglich.

Weiterhin kann der Reinigungsmittelformkörper Salze zur Regulierung der Konsistenz und des Abspülverhaltens enthalten. Im Allgemeinen handelt es sich bei diesen Salzen um anorganische Salze, die vorzugsweise aus der Gruppe der Alkali- und Erdalkalisalze der Schwefelsäuren, Phosphorsäuren, Stickstoffsäuren, Kohlensäure, Halogensäuren wie HCl oder deren Mischungen ausgewählt werden. Der Anteil an diesen Salzen im Reinigungsmittelformkörper kann bis zu 80 Gew.% betragen, vorzugsweise etwa 20 Gew.% bis 50 Gew.%. Besonders bevorzugt ist, als Salze Natriumchlorid oder Natriumsulfat oder deren Mischung einzusetzen.

Zudem kann der Reinigungsmittelformkörper Lignin oder Ligninsulfonat als Füllstoff umfassen.

Der Reinigungsmittelformkörper kann weiterhin Extrusionshilfsmittel, vorzugsweise bis zu einem Anteil von 15 Gew.%, umfassen. Als Extrusionshilfsmittel können beispielsweise Alkylpolyethylenglycolether mit bis zu 40 EO, aber auch andere bekannte Extrusionshilfsmittel, wie zum Beispiel Cellulose und ihre Derivate, verwendet werden.

Weiterhin kann der Reinigungsmittelformkörper Farbstoffe und Pigmente wie beispielsweise Titandioxid umfassen, aber optional auch Desinfektionsmittel, Konservierungsmittel, Bleichmittel, Aktivatoren, Enzyme, Komplexierungsmittel und Säuren.

Grundsätzlich ist die Reinigungsmittelformkörperphase vorzugsweise weiß, weil dadurch der Farbkontrast der verschiedenfarbigen Gelphasen besonders hervorgehoben wird. In einer Variante kann der Reinigungsmittelformkörper jedoch ebenfalls eingefärbt sein, was dem Gesamtprodukt ein sehr poppiges Aussehen verleiht.

Neben dem Tenside enthaltenden, im Allgemeinen extrudierten Reinigungsmittelformkörper kann die Reinigungsmittelformkörperphase prinzipiell auch eine Seifenphase sein. Allerdings sollte die Seifenphase dann auf jeden Fall zusätzlich zu den Alkancarbonsäuresalzen auch noch weitere Tenside umfassen, um die gewünschte Reinigungswirkung zu erzielen.

Weiterhin kann der Reinigungsmittelformkörper prinzipiell auch eine wasserlösliche oder wasserdispergierende Polymere umfassende Phase sein, die Tenside umfasst.

### III. Erfindungsgemäße Mittel

Das erfindungsgemäße stückförmige Mittel kann unterschiedlich geformt sein, beispielsweise quaderförmig.

Üblicherweise ist das Mittel so geformt, dass es in ein herkömmliches, am Rand der Toilette befestigbares Toilettenkörbchen eingebracht werden kann.

Im Allgemeinen besteht das Mittel aus dem Reinigungsmittelformkörper, dessen Anteil an dem Mittel wenigstens 10 Gew.%, vorzugsweise wenigstens 50 Gew.% und besonders bevorzugt wenigstens 60 Gew.% betragen sollte.

Der Anteil der Gelphase beträgt damit höchstens 90 Gew.%, vorzugsweise höchstens 50 Gew.% und besonders bevorzugt höchstens 40 Gew.% des Mittels.

Die Sichtseite des Mittels sollte zu einem überwiegenden Anteil aus der Gelphase bestehen, da die Gelphase ja wenigstens im Bereich der Sichtöffnungen in dem Behältnis für den Verbraucher sichtbar sein soll. Die drei anderen Seiten des Reinigungsmittels sind aus der Reinigungsmittelformkörperphase gebildet, die letztendlich für die Gelphase eine Art Wanne bildet, in die die Gelphase mit der Farbänderung über die "Breite" der Wanne eingefüllt ist. Die restlichen beiden Seiten des Mittels bestehen aus Reinigungsmittelformkörperphase und Gelphase.

Zur Herstellung des erfindungsgemäßen Mittels werden die Bestandteile des Reinigungsmittelformkörpers gemischt und in einem kontinuierlichen Verfahren zu einem im Schnitt U- oder wannenförmigen Endlosstrang extrudiert. Durch die U- bzw. Wannenform ist eine Begrenzung gegeben, so dass ein Aufnahmeraum zur Aufnahme der flüssigen Gelphase bereitgestellt wird. Gleich nach der erfolgten Extrusion werden in den Aufnahmeraum von oben über wenigstens zwei Düsen, die jeweils in den Randbereichen des Aufnahmeraums zwei verschiedenfarbige heiße Gelphasen zudosieren, die farbigen Gelmassen eingefüllt, die sich zum Teil vermischen und nach dem Erkalten zu einer glatten transparenten Gelfläche mit einem Farbverlauf aushärten.

Nach dem Erkalten werden von dem befüllten Endlosstrang stückförmige Reinigungsmittel abgeschnitten.

Die vorliegende Erfindung betrifft somit auch ein Verfahren zur Herstellung eines Reinigungsmittels, insbesondere zur Bevorratung in einer Vorrichtung nach einem der Ansprüche 1 bis 7, dadurch gekennzeichnet, dass zunächst die Reinigungsmittelformkörperphase in U-Form extrudiert wird, wodurch an der Oberseite der Reinigungsmittelformkörperphase ein Aufnahmeraum gebildet wird und wenigstens zwei Düsen vorgesehen sind, die sich oberhalb des Aufnahmeraums nahe der beiden Schenkel des Reinigungsmittelformkörpers befinden, und durch diese beiden Düsen zwei verschiedenfarbige heiße Gelphasen in den Aufnahmeraum gespritzt werden, um sich dort teilweise zu vermischen und zu erkalten.

Die Erfindung sowie weitere vorteilhafte Ausführungsformen und Weiterbildungen derselben werden im Folgenden anhand der in den Zeichnungen dargestellten Beispiele näher beschrieben und erläutert. Die der Beschreibung und den Zeichnungen zu entnehmenden Merkmale können einzeln für sich oder zu mehreren in beliebiger Kombination erfindungsgemäß angewandt werden. Es zeigen:
- Figur 1: eine schematische Ansicht einer erfindungsgemäßen Vorrichtung zur Aufnahme des Reinigungsmittels,

- Figur 2: eine perspektivische Ansicht des erfindungsgemäßen Mittels von schräg oben mit angedeutetem Farbverlauf,
- Figur 2a: den schematischen örtlichen Verlauf der Extinktion z.B. bei der Wellenlänge 480 nm,
- Figur 3: die Vorrichtung aus Figur 1, in die schematisch dargestellt, das erfindungsgemäße Reinigungsmittel aus Figur 2 mit dem Farbverlauf eingefügt ist,
- Figur 4: eine Variante der Form der Reinigungsmittelformkörperphase im Schnitt,
- Figur 5: eine weitere Variante der Form der Reinigungsmittelformkörperphase im Schnitt,
- Figur 6: eine schematische Darstellung des eingehängten erfindungsgemäßen Körbchens mit dem Mittel in einer Toilettenschüssel,
- Figur 7: ein Beispiel eines WC-Körbchens mit vier blumenförmigen Sichtöffnungen und dem Mittel und
- Figur 8: ein Beispiel für ein WC-Körbchen mit den verschiedengroßen, runden Öffnungen.

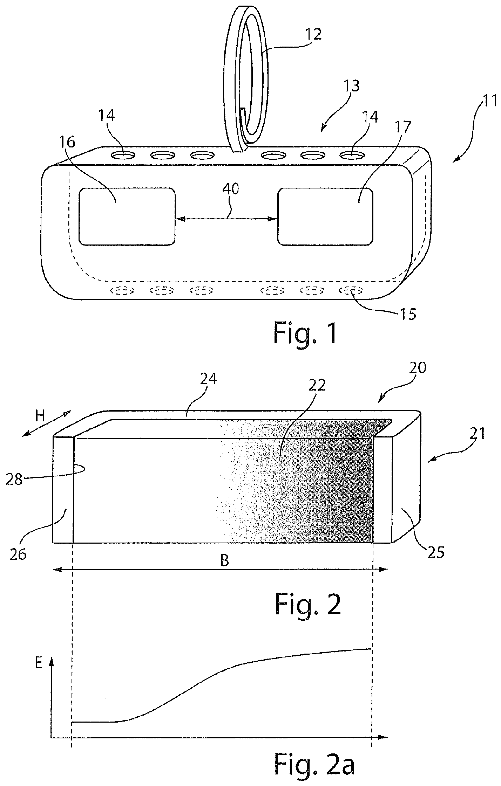

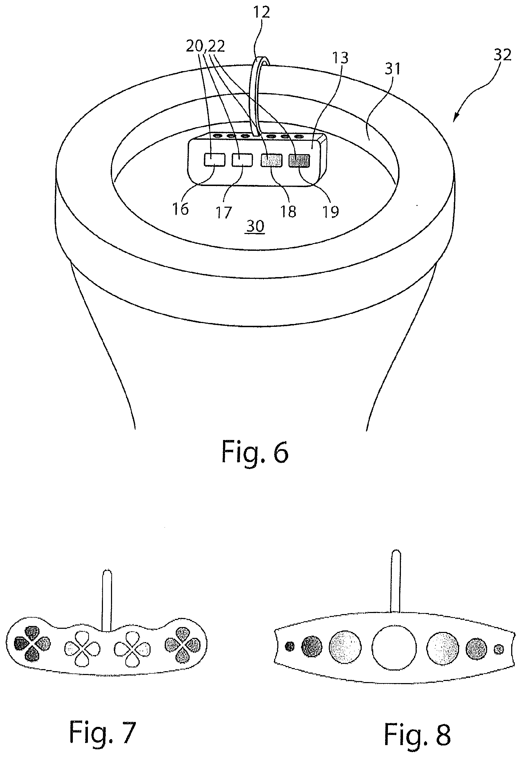

Bei der erfindungsgemäßen Vorrichtung 11 handelt es sich um ein Toilettenkörbchen 11 mit einer Haltevorrichtung 12, die dazu dient, den Rand 31 einer Toilettenschüssel 32 zu umgreifen und einem Behältnis 13, das zur Bevorratung von Toilettenreinigungsmittel 20 dient, vgl. Fig. 1 und Fig. 6. Weiterhin weisen solche Behältnisse 13 üblicherweise Zuflussöffnungen 14 für den Zutritt des Spülwassers in das Behältnis 13 und Abflussöffnungen 15 auf, durch die das Spülwasser mit einem Teil des gelösten Reinigungsmittels 20 in die Toilettenschüssel 32 gespült wird. Ein solcher Aufbau ist an sich bekannt.

Die erfindungsgemäße Vorrichtung 11 weist nun wenigstens zwei in Richtung des Inneren 30 der Toilettenschüssel 32 weisende Sichtöffnungen 16, 17 auf, die voneinander beabstandet sind, vgl. Fig. 1 und Fig. 6. Durch diese Sichtöffnungen 16, 17 ist das in dem Behältnis 13 befindliche Reinigungsmittel 20, nämlich dessen Gelphase 22, gut sichtbar. Nachdem die beiden Sichtöffnungen 16, 17 voneinander beabstandet sind, wirken die beiden Sichtöffnungen 16, 17 wie getrennte Fenster und lassen jeweils einen anderen Bereich des in dem Behältnis 13 befindlichen Reinigungsmittels 20 beziehungsweise dessen Gelphase 22 erscheinen.

Eine jede Sichtöffnung 16, 17, 18, 19 sollte eine Breite zwischen 0,2 cm und 5 cm, vorzugsweise zwischen 0,5 und 2 cm aufweisen, wobei die Sichtöffnungen 16, 17, 18, 19 quadratisch, rund, oval, sternförmig, tropfenförmig oder anders geformt sein können. Auch Motive wie Tiermotive oder geometrische Formen sind als Form der Sichtöffnungen 16, 17, 18, 19 möglich.

Zudem müssen die Sichtöffnungen 16, 17, 18, 19 auch nicht alle gleichartig geformt sein, auch verschiedene unterschiedliche Öffnungsformen sind möglich.

Das Toilettenkörbchen 11 kann weiß, farbig, milchig oder auch transparent sein.

Die Sichtöffnungen 16, 17, 18, 19 sollten voneinander beabstandet sein, in der Regel sollte die Stegbreite 40 zwischen zwei Sichtöffnungen wenigstens 1 mm, vorzugsweise wenigstens 2 mm und weniger als 30 mm, vorzugsweise weniger als 20 mm betragen. Selbstverständlich können die Abstände zwischen zwei Sichtöffnungen unterschiedlich sein.

Das erfindungsgemäße Mittel 20 ist in **Figur 2** dargestellt. Es weist eine Reinigungsmittelformkörperphase 21, die Tenside umfasst und der Reinigung dient, und eine Gelphase 22, die Gelbildner, Duftstoffe und Lösemittel umfasst und der Beduftung dient, auf.

Die Reinigungsmittelformkörperphase 21 ist im Schnitt U-förmig ausgebildet und besteht aus einem Boden 24 und einem rechten 25 und einem linken 26 Schenkel. Der Boden 24 und die beiden Schenkel 25, 26 begrenzen den Aufnahmeraum 28 für die Gelphase 22. In Figur 2 ist die Reinigungsmittelformkörperphase 21 nach vorne gekippt, so dass man auf die Sichtseite draufsieht.

Die Reinigungsmittelformkörperphase 21 wird vorzugsweise als U-förmiger Endlosstrang extrudiert, wobei der Aufnahmeraum 28 während des Herstellungsprozesses nach oben weist.

In diesen Aufnahmeraum 28 werden dann über wenigstens zwei Düsen, wobei eine Düse nahe dem rechten Schenkel 25 und eine Düse nahe dem linken Schenkel 26 des Reinigungsmittelformkörpers 21 angeordnet ist, zwei heiße flüssige Gelphasen 22 unterschiedlicher Farbe gefüllt, die dann im Mittenbereich des Aufnahmeraums 28 ineinanderlaufen, wohingegen in den Bereichen nahe der Schenkel 25, 26 jeweils quasi die reine Gelphase 22 der jeweiligen Farbe erstarrt. Auf diese Weise erhält man einen kontinuierlichen ansprechenden Farbverlauf in der Gelphase 22 vom rechten Schenkel 25 der Reinigungsmittelformkörperphase 21 zum linken Schenkel 26 der Reinigungsmittelformkörperphase 21.

Die zwischen den Innenseiten der beiden Schenkel 25, 26 gemessene Breite B der Gelphase 22 kann zwischen 1 cm und 20 cm, vorzugsweise zwischen 3 cm und 15 cm betragen. Um bei einer nicht allzu hohen Farbstoffkonzentration einen frischen kräftigen Farbeindruck zu vermitteln, sollte die Gelphase 22 wenigstens 0,5 mm, vorzugsweise wenigstens 2 mm dick beziehungsweise hoch sein. Je dicker die Schichtdicke der Gelphase 22 ist, desto leichter lässt sich auch eine ebene Gelphase 22 mit einem ansprechenden Farbverlauf herstellen, da eine dickere Schicht einem schnelleren Erkalten und damit verbundenen Schlieren an der Oberfläche entgegenwirkt.

Die Schichtdicke der Reinigungsmittelformkörperphase 21 hängt auch von der zu erzielenden Reinigungswirkung und den gewünschten Spülzahlen bei der jeweiligen Geometrie ab. Vorzugsweise ist der Reinigungsmittelformkörper 21 zwischen 3 mm und 30 mm, vorzugsweise zwischen 5 mm und 20 mm dick.

Die Höhe H des Mittels 20 beträgt üblicherweise zwischen 2 mm und 50 mm, vorzugsweise zwischen 5 mm und 30 mm, so dass das Mittel 20 noch gut unterhalb des Spülrands 31 der Toilette 32 positioniert werden kann.

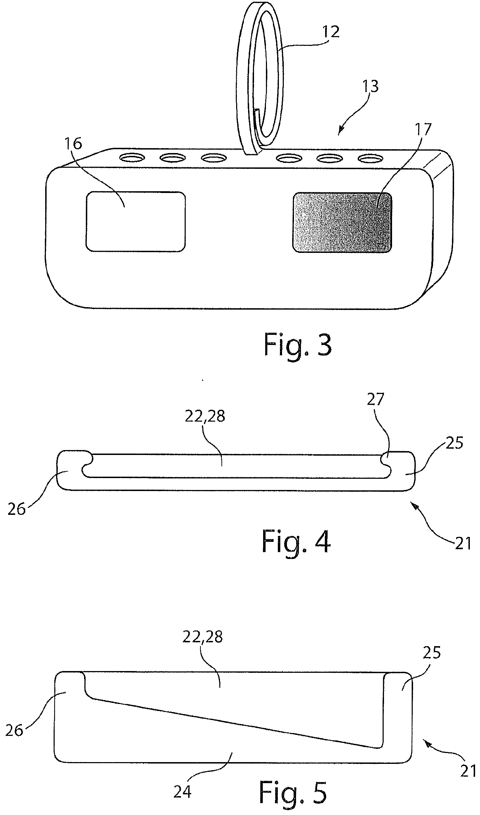

Das Reinigungsmittel 20 gemäß Figur 2, das in ein WC-Körbchen 11 aus Figur 1 angeordnet ist, ist in **Figur 3** dargestellt.

Durch die linke Sichtöffnung 16 in **Figur 3** erkennt man den Bereich des Mittels 20 mit der Farbe der nahe dem linken Schenkel 26 eingefüllten Gelphase 22, in der Sichtöffnung 17 dominiert die Farbe der Gelphase, die nahe dem rechten Schenkel 25 eingefüllt wurde. Diese verschiedenen Farben sind in den Figuren 2 und 3 schematisch durch einen weißgrauen Farbverlauf dargestellt.

Nachdem die beiden Farben sich in Richtung der Mitte des Aufnahmeraums 28 durchmischen, korrespondieren die beiden durch die Sichtfenster 16, 17 hindurchscheinenden farbigen Flächen auch sehr harmonisch, da eine jede Farbfläche auch Anteile der anderen Farbe aufweist. Der Farbverlauf innerhalb eines jeden Sichtfensters 16, 17 trägt zu einer interessanten, ungewöhnlichen Optik bei.

In **Figur 4** und **5** sind weitere mögliche im Wesentlichen U-förmige Reinigungsmittelformkörperphasen 21 im Schnitt dargestellt. Wie in Figur 4 dargestellt, können die Schenkel 25, 26 an ihrer nach oben weisenden Seite Nasen 27 aufweisen, die einen ansprechenden optischen Abschluss für die Gelphase 22 bilden und ein direktes Eindringen des Spülwassers in den Phasenübergang Gelphase 22/Reinigungsmittelformkörperphase 21 und ein Herausfallen der Gelphase 22 verhindern.

Auch kann der Boden 24 der Reinigungsmittelformkörperphase 21 beispielsweise an der Innenseite des Aufnahmeraums 28 gemäß Figur 5 schräg verlaufen, wodurch bei einer rein weißen Reinigungsmittelformkörperphase 21 eine Aufhellung der Farbe der Gelphase 22 in dem Bereich mit der dünneren Gelphasenschicht 22 erzielt wird.

Auch andere Formen der Reinigungsmittelformkörperphase 21 sind möglich. Vorzugsweise sollten allerdings die Außenseite des Bodens 24 und der beiden Schenkel 25, 26 senkrecht zueinander stehen, um eine einfache Extrusion eines Endlosstrangs zu ermöglichen.

Selbstverständlich kann die Reinigungsmittelformkörperphase 21 ebenfalls angefärbt sein.

Beispiele für ansprechend geformte Toilettenkörbchen mit vier blumenförmigen jeweils aus vier Einzelblättern bestehenden Sichtöffnungen sind in Figur 7 und mit sieben runden Sichtöffnungen verschiedener Größe in Figur 8 dargestellt. In den Figuren 7 und 8 verändert sich die Farbe der Gelphase von rot über gelb nach grün.

Nachfolgend ist beispielhaft eine Rahmenrezeptur für die Reinigungsmittelformkörperphase 21 und die Gelphase 22 angegeben:

### Gelphase

| Rohstoff: | Funktion | Anteil (Gew.%) |
|---|---|---|
| Fettalkohol (C9-C15) Ethoxylate mit 10 oder 11 EO | nicht ionisches Tensid | <50 |
| Natrium-Dodecylbenzolsulfonat 80 %ig + Toluolsulfonat | anionisches Tensid | <2 |
| Natriumstearat gesprüht | anionisches Tensid | <5 |
| Wasser | Lösemittel | < 10 |
| 1,2 Propandiol | Lösemittel | < 10 |
| Gelborangefarbstoff | Farbstoff 1 | > 0,05 |
| Rotfarbstoff | Farbstoff 2 | > 0,02 |
| Duft | | > 30 |

### Reinigungsmittelformkörperphase

| Rohstoff: | Funktion | Anteil (Gew.%) |
|---|---|---|
| Natrium-Dodecylbenzolsulfonat, 90 %ig | anionisches Tensid | < 20 |
| Cumolsulfonat, Na-Salz 93 %ig | Hydrothop | < 10 |
| α-Olefinsulfonat, Na-Salz | anionisches Tensid | < 10 |
| Natriumsulfat | Salz | < 50 |
| Natriumchlorid | Salz | < 30 |
| Polyethylenglykol 6000 DS | Stellmittel | < 5 |
| Titandioxid | Farbstoff 3 | < 0,02 |

## Patentansprüche

1. Vorrichtung (11) mit stückförmigem Reinigungsmittel (20) für den Toilettenbereich mit einer Haltevorrichtung (12), die am Rand (31) einer Toilettenschüssel (32) befestigbar ist und einem Behältnis (13), das das Reinigungsmittel (20) mit einer Reinigungsmittelformkörperphase (21) und einer Gelphase (22) umfasst, wobei das Reinigungsmittel (20) dazu dient, Duftstoffe freizugeben und sich beim Überspülen mit Wasser nach und nach aufzulösen,
**dadurch gekennzeichnet, dass** das Behältnis (13) wenigstens zwei in Richtung des Inneren (30) der Toilettenschüssel (32) weisende, voneinander beabstandete Sichtöffnungen (16, 17) aufweist, durch die die Gelphase (22) sichtbar ist, und sich die Farbe der Gelphase (22) von der einen Seite des Reinigungsmittels (20) zu der anderen Seite des Reinigungsmittels (20) im Wesentlichen kontinuierlich ändert, so dass sich der Farbeindruck der Gelphase (22) hinter der ersten Sichtöffnung (16) von dem Farbeindruck der Gelphase (22) hinter der zweiten Sichtöffnung (17) unterscheidet.

2. Vorrichtung (11) nach Anspruch 1, **dadurch gekennzeichnet, dass** sich das Mittel (20) beziehungsweise dessen Gelphase (22) wenigstens über den gesamten Bereich der Öffnungen (16) und (17) erstreckt.

3. Vorrichtung (11) nach einem der Ansprüche 1 oder 2, **dadurch gekennzeichnet, dass** die Gelphase (22) im Wesentlichen transparent und die Reinigungsmittelformkörperphase (21) nicht transparent ist.

4. Vorrichtung (11) nach einem der vorangegangenen Ansprüche, **dadurch gekennzeichnet, dass** die Reinigungsmittelformkörperphase (21) einen Aufnahmeraum (28) für die Gelphase (22) bildet, der im Schnitt vorzugsweise im Wesentlichen U-förmig oder wannenförmig ist.

5. Vorrichtung (11) nach einem der vorangegangenen Ansprüche, **dadurch gekennzeichnet, dass** die Gelphase (22) Gelbildner, Duftstoffe und Lösemittel aufweist und/oder die Reinigungsmittelformkörperphase (21) Tenside umfasst.

6. Vorrichtung (11) nach einem der vorangegangenen Ansprüche, **dadurch gekennzeichnet, dass** die Vorrichtung (11) wenigstens drei (16, 17, 18), vorzugsweise wenigstens vier Sichtöffnungen (16, 17, 18, 19) aufweist und/oder das Reinigungsmittel (20) wenigstens drei, vorzugsweise wenigstens vier verschiedene, im Wesentlichen kontinuierlich ineinander übergehende Farben.

7. Vorrichtung (11) nach einem der vorangegangenen Ansprüche, **dadurch gekennzeichnet, dass** die Sichtöffnungen (16, 17, 18, 19) des Behältnisses (13) jeweils zwischen 0,2 cm und 5 cm, vor-zugsweise zwischen 0,5 und 2 cm breit sind und/oder die Sichtöffnungen (16, 17, 18, 19) rechteckig, rund, stern-, tropfenförmig oder oval sind.

8. Vorrichtung (11) nach einem der vorangegangenen Ansprüche, **dadurch gekennzeichnet, dass** der Anteil von Duftstoffen in der Gelphase (22) vorzugsweise wenigstens 2 Gew.%, vorzugsweise zwischen 6 Gew.% und 50 Gew.% und besonders bevorzugt zwischen 15 Gew.% und 35 Gew.% beträgt.

9. Vorrichtung (11) nach einem der vorangegangenen Ansprüche, **dadurch gekennzeichnet, dass** der Anteil des Farbstoffs in den Gelphasen (22) bis zu 10 Gew.%, vorzugsweise 0,001 Gew.% bis 5 Gew.% und besonders bevorzugt zwischen 0,01 Gew.% und 0,1 Gew.% beträgt.

10. Vorrichtung (11) nach einem der vorangegangenen Ansprüche, **dadurch gekennzeichnet, dass** der Schmelzpunkt der Gelphase (22) vorzugsweise wenigstens 40 °C, bevorzugt wenigstens 50 °C beträgt.

11. Vorrichtung (11) nach einem der vorangegangenen Ansprüche, **dadurch gekennzeichnet, dass** der Anteil des Reinigungsmittelformkörpers (21) an dem Mittel (20) wenigstens 10 Gew.%, vorzugsweise wenigstens 50 Gew.% und besonders bevorzugt wenigstens 60 Gew.% beträgt.

12. Verfahren zur Herstellung eines Reinigungsmittels (20), insbesondere zur Bevorratung in einer Vorrichtung (11) nach einem der Ansprüche 1 bis 11, **dadurch gekennzeichnet, dass** zunächst die Reinigungsmittelformkörperphase (21) in U-Form extrudiert wird, wodurch an der Oberseite der Reinigungsmittelformkörperphase (21) ein Aufnahmeraum (28) gebildet wird und wenigstens zwei Düsen vorgesehen sind, die sich oberhalb des Aufnahmeraums (28) nahe der beiden Schenkel (25, 26) des Reinigungsmittelformkörpers (21) befinden, und durch diese beiden Düsen zwei verschiedenfarbige heiße Gelphasen (22) in den Aufnahmeraum (28) gespritzt werden, um sich dort teilweise zu vermischen und zu erkalten.

## Claims

1. A device (11) having a block-shaped cleaning agent (20) for the toilet area, a retaining device (12) which may be fastened to a rim (31) of a toilet bowl (32), and a container (13) comprising said cleaning agent (20) having a cleaning agent shaped-body phase (21) and a gel phase (22), said cleaning agent (20) serving the purpose of releasing fragrances and of gradually dissolving when flushed with water, **characterised in that** the container (13) has at least two viewing openings (16, 17) facing in the direction of the interior (30) of the toilet bowl (32) which are spaced apart from each other and through which the gel phase (22) is visible, and **in that** the colour of the gel phase (22) changes in an essentially continuous manner from one side of the cleaning agent (20) to the other side of the cleaning agent (20), such that the colour of the gel phase (22) perceived behind the first viewing opening (16) will be different from the colour of the gel phase (22) perceived behind the second viewing opening (17) .

2. The device (11) as claimed in claim 1, **characterised in that** the agent (20), or the gel phase (22) thereof, extends over at least the entire surface areas of the openings (16) and (17).

3. The device (11) as claimed in any one of claims 1 or 2, **characterised in that** the gel phase (22) is essentially transparent and the cleaning agent shaped-body phase (21) is not transparent.

4. The device (11) as claimed in any of the preceding claims, **characterised in that** the cleaning agent shaped-body phase (21) forms a retention space (28) for holding the gel phase (22) the cross-section of which is essentially U-shaped or trough-shaped.

5. The device (11) as claimed in any of the preceding claims, **characterised in that** the gel phase (22) has gel-forming agents, fragrances and solvents, and/or the cleaning agent shaped-body phase (21) comprises surface-active agents.

6. The device (11) as claimed in any of the preceding claims, **characterised in that** the device (11) has at least three (16, 17, 18), preferably at least four viewing openings (16, 17, 18, 19), and/or **in that** the cleaning agent (20) has at least three, preferably at least four different colours merging into one another in an essentially continuous manner.

7. The device (11) as claimed in any of the preceding claims, **characterised in that** the viewing openings (16, 17, 18, 19) of the container (13) have each a width of between 0.2 cm and 5 cm, preferably between 0.5 cm and 2 cm, and/or **in that** said viewing openings (16, 17, 18, 19) are rectangular, round, star-shaped, drop-shaped or oval.

8. The device (11) as claimed in any of the preceding claims, **characterised in that** the proportion of fragrances present in the gel phase (22) is preferably at least 2 % by weight, preferably between 6 % by weight and 50 % by weight, and most preferably between 15 % by weight and 35 % by weight.

9. The device (11) as claimed in any of the preceding claims, **characterised in that** the proportion of colouring agent present in the gel phases (22) is up to 10 % by weight, preferably between 0.001 % by weight and 5 % by weight, and most preferably between 0.01 % by weight and 0.1 % by weight.

10. The device (11) as claimed in any of the preceding claims, **characterised in that** the melting point of the gel phase (22) is preferably at least 40 °C, more preferably at least 50 °C.

11. The device (11) as claimed in any of the preceding claims, **characterised in that** the proportion of the cleaning agent shaped body (21) present in the agent (20) is at least 10 % by weight, preferably at least 50 % by weight, and most preferably at least 60 % by weight.

12. A method of fabricating a cleaning agent (20), in particular for storing in a device (11) as claimed in any one of claims 1 to 11, **characterised in that** initially the cleaning agent shaped-body phase (21) is extruded in a U-shaped form such that on the top surface of said cleaning agent shaped-body phase (21) a retention space (28) is formed, and **in that** at least two nozzles are provided which are located above said retention space (28) near the two legs (25, 26) of the cleaning agent shaped body (21), and **in that** through these two nozzles, two differently coloured, hot gel phases (22) are injected into the retention space (28) so as to partially become mixed and to cool off therein.

## Revendications

1. Dispositif (11) contenant un produit de nettoyage (20) en forme de pain pour la zone des toilettes, pourvu d'un dispositif d'attache (12) qui peut être fixé au rebord (31) d'une cuvette de toilettes (32) et d'un récipient (13) comprenant le produit de nettoyage (20) qui présente une phase de corps moulé de produit de nettoyage (21) et une phase de gel (22), le produit de nettoyage (20) servant à délivrer des substances odorantes et se dissolvant peu à peu à chaque passage de l'eau de la chasse, **caractérisé en ce que** le récipient (13) présente au moins deux ouvertures de visualisation (16, 17) espacées entre elles et tournées vers l'intérieur (30) de la cuvette de toilettes (32) à travers lesquelles la phase de gel (22) est visible et **en ce que** la couleur de la phase de gel (22) change essentiellement de manière continue depuis un côté du produit de nettoyage (20) vers l'autre côté du produit de nettoyage (20) de sorte que la couleur de la phase de gel (22) perçue derrière la première ouverture de visualisation (16) est différente de la couleur de la phase de gel (22) perçue derrière la deuxième ouverture de visualisation (17).

2. Dispositif (11) selon la revendication 1, **caractérisé en ce que** le produit (20), à savoir dans sa phase de gel (22), s'étend au moins sur toute la zone des ouvertures (16) et (17).

3. Dispositif (11) selon l'une quelconque des revendications 1 ou 2, **caractérisé en ce que** la phase de gel (22) est essentiellement transparente et que la phase de corps moulé de produit de nettoyage (21) n'est pas transparente.

4. Dispositif (11) selon l'une quelconque des revendications précédentes, **caractérisé en ce que** la phase de corps moulé de produit de nettoyage (21) forme un espace de réception (28) pour la phase de gel (22) lequel est, en coupe transversale, de préférence essentiellement en forme de U ou en forme de cuve.

5. Dispositif (11) selon l'une quelconque des revendications précédentes, **caractérisé en ce que** la phase de gel (22) présente des gélifiants, des substances odorantes et des solvants et/ou que la phase de corps moulé de produit de nettoyage (21) comprend des tensioactifs.

6. Dispositif (11) selon l'une quelconque des revendications précédentes, **caractérisé en ce que** ledit dispositif (11) présente au moins trois (16, 17, 18), de préférence au moins quatre ouvertures de visualisation (16, 17, 18, 19) et/ou que le produit de nettoyage (20) présente au moins trois, de préférence au moins quatre couleurs différentes qui se fondent essentiellement de manière continue les unes dans les autres.

7. Dispositif (11) selon l'une quelconque des revendications précédentes, **caractérisé en ce que** les ouvertures de visualisation (16, 17, 18, 19) du récipient (13) présentent respectivement une largeur comprise entre 0,2 cm et 5 cm, de préférence entre 0,5 et 2 cm, et/ou que les ouvertures de visualisation (16, 17, 18, 19) sont rectangulaires, rondes, étoilées, en forme de goutte ou ovales.

8. Dispositif (11) selon l'une quelconque des revendications précédentes, **caractérisé en ce que** la proportion de substances odorantes contenue dans la phase de gel (22) est de préférence d'au moins 2 % poids, est comprise de préférence entre 6 % poids et 50 % poids et est comprise de manière particulièrement privilégiée entre 15 % poids et 35 % poids.

9. Dispositif (11) selon l'une quelconque des revendications précédentes, **caractérisé en ce que** la proportion de matière colorante contenue dans les phases de gel (22) fait jusqu'à 10 % poids, est comprise de préférence entre 0,001 % poids et 5 % poids et est comprise de manière particulièrement privilégiée entre 0,01 % poids et 0,1 % poids.

10. Dispositif (11) selon l'une quelconque des revendications précédentes, **caractérisé en ce que** le point de fusion de la phase de gel (22) est de préférence d'au moins 40 °C, de manière privilégiée d'au moins 50 °C.

11. Dispositif (11) selon l'une quelconque des revendications précédentes, **caractérisé en ce que** la proportion du corps moulé de produit de nettoyage (21) contenue dans le produit (20) est d'au moins 10 % poids, de préférence d'au moins 50 % poids et de manière particulièrement privilégiée d'au moins 60 % poids.

12. Procédé destiné à fabriquer un produit de nettoyage (20), en particulier pouvant être stocké dans un dispositif (11) selon l'une quelconque des revendications 1 à 11, **caractérisé en ce que** la phase de corps moulé de produit de nettoyage (21) est d'abord extrudée en forme de U, ce qui forme un la phase de corps moulé de produit de nettoyage (21), et **en ce qu'**au moins deux buses sont prévues, lesquelles se trouvent au-dessus de l'espace de réception (28), à proximité des deux côtés (25, 26) du corps moulé de produit de nettoyage (21) et **en ce que** deux phases de gel (22) chaudes de couleurs différentes sont injectées par lesdites deux buses dans l'espace de réception (28) afin de s'y mélanger partiellement et de refroidir.
